Europäisches Patentamt

European Patent Office

Office européen des brevets

(19)

(11) Veröffentlichungsnummer: **0 209 706**
**B1**

(12) EUROPÄISCHE PATENTSCHRIFT

(45) Veröffentlichungstag der Patentschrift: **14.03.90**

(51) Int. Cl.⁵: **H 01 T 23/00, A 61 N 1/44**

(21) Anmeldenummer: **86107965.5**

(22) Anmeldetag: **11.06.86**

(54) Ionenerzeuger.

(30) Priorität: **20.07.85 DE 3526021**

(43) Veröffentlichungstag der Anmeldung:
**28.01.87 Patentblatt 87/05**

(45) Bekanntmachung des Hinweises auf die
Patenterteilung:
**14.03.90 Patentblatt 90/11**

(84) Benannte Vertragsstaaten:
**AT BE CH DE FR GB IT LI LU NL SE**

(56) Entgegenhaltungen:
**DE-A-3 021 900**
**DE-B-1 281 602**
**DE-U-8 521 014**
**US-A-3 818 269**
**US-A-4 173 229**

(73) Patentinhaber: **HOFMANN & VÖLKEL GMBH**
**Königsallee 4**
**D-8540 Bayreuth (DE)**

(72) Erfinder: **Völkel, Helmut**
**Grüner Baum 2**
**D-8580 Bayreuth (DE)**

(74) Vertreter: **Jaeger, Klaus, Dr. et al**
**Patentanwälte Jaeger-Steffens-Köster-Lorenz**
**Pippinplatz 4a Postfach 1620**
**D-8035 Gauting (DE)**

Courier Press, Leamington Spa, England.

## Beschreibung

Die Erfindung betrifft einen tragbaren Ionenerzeuger mit einer in einem Gehäuse angeordneten Gleichspannungs-Hochspannungsquelle mit zwei Ausgängen, die eine aus dem Gehäuse vorstehende nadelförmige Hochspannungselektrode aufweist, die elektrisch leitend mit einem Ausgang verbunden ist.

Ein derartiger Ionenerzeuger ist aus der DE-A-28 22 228 bekannt. Bei diesem bekannten Ionenerzeuger, der als Anti-Static-Einrichtung eingesetzt wird, wird eine periodische Folge separater positiver oder negativer Ladungsimpulse erzeugt, welche die umgebende Luft ionisieren. Aus dem Gehäuse dieses Ionenerzeugers ragt die mit der Hochspannungsquelle elektrisch leitend verbundene Hochspannungselektrode vor. Die auf dem zur Hochspannungselektrode entgegengesetzten elektrischen Potential liegende Gegenelektrode ist dort einfach durch das Massepotential der elektrischen Schaltung zur Erzeugung der Hochspannung gebildet. Diese nicht speziell ausgebildete Gegenelektrode befindet sich also im Inneren des Gehäuses, so dass sich im eingeschalteten Zustand dieser bekannten Einrichtung zwischen der nadelförmigen Hochspannungselektrode und dem Gehäuse ein elektrisches Feld ausbildet Diese bekannte Anti-Static-Einrichtung wird zur Entladung statischer Elektrizität auf Schallplatten verwendet. Sie kann auch zur Entladung statischer Elektrizität auf Papieren, die in elektrostatischen Photokopierern verwendet werden, zur Entladung statischer Elektrizität von Etiketten auf Kunststoff-Flaschen, und zum Auftragen von Farb- und Übertragungs-Medien auf Kunststoff-Fahrzeugkarosserien verwendet werden.

Aus der DE-B-25 21 179 ist ein Ionenerzeuger bekannt, der mit einer an Netzwechselspannung anzulegenden Gasentladungslampe, insbesondere mit einer Glimmlampe, versehen ist. Deshalb ist dieser Ionenerzeuger nur in Verbindung mit einem Netzanschluss als quasi ortsfester Ionenerzeuger verwendbar. Da die Ionenerzeugungsrate mit der Entfernung von dem Ionenerzeuger überproportional abnimmt, ist die Wirksamkeit eines solchen stationären Ionenerzeugers nur in seiner unmittelbaren Nachbarschaft zufriedenstellend, während seine Wirksamkeit in einiger Entfernung bereits Wünsche übrig lässt Zur Verbesserung seiner Wirksamkeit in grösserer Entfernung kann ein derartiger Ionenerzeuger mit einem Gebläse ausgebildet sein, mit welchem die erzeugten Ionen eine grössere Wegstrecke transportiert werden können Durch die Ausbildung des Ionenerzeugers mit einem Gebläse ergibt sich jedoch nicht nur ein erheblicher baulicher Aufwand, sondern auch eine bestimmte Mindestgrösse eines derartigen Ionenerzeugers.

Ein Ionenerzeuger zur Ionisierung von Raumluft und zur Ionisierung anderer Gase mit einem in einem Gehäuse angeordneten Hochspannungstransformator und mit zwei Gleichrichtern ist aus der DE-C-25 35 621 bekannt. Bei diesem Ionisator ist an dem Hochspannungstransformator ein Rohrstutzen angeordnet, an dem wahlweise eine negative oder eine positive Spannung angelegt werden kann. Auf den Rohrstutzen kann ein Sprühnadelaggregat zur Ionisierung der Raumluft, bzw. ein Behälter für ein Gas aufgesteckt werden, der in seinem Inneren ebenfalls mit einem Sprühnadelaggregat versehen sein kann. Die zuletzt genannte Ausführungsform eines Ionenerzeugers mit einem Behälter dient insbesondere dazu, eine Injektionsspritze mit positiv oder negativ ionisiertem Gas zu füllen und mit der mit ionisiertem Gas gefüllten Injektionsspritze intrakutane Injektionen vorzunehmen. Diese intrakutanen Injektionen dienen dazu, feststellen zu können, auf welche Polarität des injizierten ionisierten Gases ein Patient anspricht (= Hautimpftest). Da dieser Ionenerzeuger gemeinsam mit einer Gasflasche verwendet werden soll, ist davon auszugehen, dass es sich auch bei diesem Ionenerzeuger um ein ortsfestes Gerät handelt.

Ein Ionenerzeuger mit einer Vorrichtung zur Erzeugung eines Luftstromes und mit einer Einrichtung zur Erzeugung von Ionen, die aus einem an einer hohen Gleichspannung liegenden Rohr besteht, sowie mit wenigstens einem dem Rohr luftseitig nachgeschalteten, auf einem hohen Negativpotential liegenden Gitter zum Auffangen von ggf. vorhandenen Ionen der entgegengesetzten Polarität, ist aus der DE-B-27 33 729 bekannt. Infolge der Verwendung einer Vorrichtung zur Erzeugung eines Luftstromes, die insbesondere als Gebläse ausgebildet ist, handelt es sich auch bei diesem Ionenerzeuger um ein ortsfestes Gerät, mit dem es möglich ist, in einem Raum eine das Wohlbefinden einer Person erhöhende Atmosphäre herzustellen.

Aus der CH-A-624 302 ist ein Gerät zum Erzeugen eines Gleichspannungsfeldes im menschlichen Körper bekannt. Dieses Gerät weist ein Gehäuse mit einer Hochspannungsquelle auf. Die Hochspannungsquelle weist eine Hochspannungselektrode und eine Gegenelektrode auf. Die Hochspannungselektrode ist als Tragelement ausgebildet, mit dem das Gerät am menschlichen Körper getragen werden kann. Vorzugsweise ist das Tragelement als Halskette ausgebildet. Die Gegenelektrode ist bei diesem Gerät dadurch elektrisch isoliert angeordnet, dass sie im Inneren des Gehäuses des Gerätes vorgesehen und in Kunststoff eingegossen ist. Bei diesem Gerät liegt das Tragelement in Form einer Halskette auf dem einen Potential der Hochspannungsquelle, d.h. auf dem Potential der Hochspannungselektrode, so dass sich im Bereich innerhalb der Halskette ein von der Gegenelektrode im Gehäuse ausgehendes elektrisches Feld ergibt, mit dem jedoch keine Ionisierung der Raumluft erfolgt, sondern mit dem infolge des den Körper durchdringenden elektrischen Feldes das Wohlbefinden der dieses Gerät tragenden Person erhöht werden soll.

Eine Vorrichtung zur Erzeugung eines ionisierten Luftstrahles ist aus der DE-B-12 81 602 bekannt. Diese Vorrichtung weist ein eine leitende Nadel umgebendes Gehäuse auf, wobei ein leitendes Ringstück an dem Gehäuse an der Ausblasöffnung der Vorrichtung angeordnet ist und diese Öffnung so überragt, dass zwischen der leitenden Nadel und der Ausblasöffnung durch eine Hochspannung erzeugte elektrische Entladung ein im Gehäuse konisch nach außen sich erweiterndes Volumenelement umfaßt. Dort liegt die leitende Nadel in der Achse der Bohrung des Gehäuses und ist zwischen der Nadel und dem leitenden Ringstück eine aus Isolierstoff bestehende Hülse vorgesehen, die sich an der Ausblasöffnung nach außen konisch fortsetzt. Diese Vorrichtung wird mit einer Druckluftquelle verbunden, um durch die Ausblasöffnung hindurch einen ausreichend schnellen Luftstrahl zu erzeugen. Mit dieser bekannten Vorrichtung ist es möglich, statische Aufladungen von einem Gegenstand zu entfernen und gleichzeitig von der Oberfläche des Gegenstandes elektrostatisch angezogene Staubteilchen wegzublasen. Außerdem können mit dieser Vorrichtung statische Entladungen sowohl an den Verunreinigungen als auch an dem zu reinigenden Gegenstand neutralisiert werden.

Aus der US-A-3 818 269 ist ein als Tischgerät ausgebildeter Ionisiator bekannt, bei dem die nadelförmige Hochspannungs-Elektrode im Bereich ihres Fußpunktes von einem parabolförmigen Reflektor umgeben ist. Dieser Reflektor liegt auf dem gleichen Potential wie die nadelförmige Hochspannungselektrode und hat den Zweck, infolge seiner gleichen Ladung wie die nadelförmige Hochspannungselektrode die emittierten Teilchen zu fokussieren und zu beschleunigen. Ein Feld entsteht dabei zwischen nadelförmiger Hochspannungselektrode und Reflektor nicht. Außerdem ist bei dem Ionisator nach der US-A-3 818 269 die nadelförmige Hochspannungselektrode von einem als Begrenzung einen Ring aufweisenden Käfig umgeben. Dieser Käfig hat jedoch lediglich den Zweck, einen rein mechanischen Schutz gegen Berührung der nadelförmigen Hochspannungselektrode zu gewährleisten. Er besteht aus isolierendem Material.

Der Erfindung liegt die Aufgabe zugrunde, einen tragbaren Ionenerzeuger der eingangs genannten Art zu schaffen, der von einem Netzanschluß und damit ortsunabhängig ist, und mit dem die zu den Atmungsorganen strömenden Partikel wie Staub, Pflanzenpollen und dergl. ionisiert und an die Körper-Außenoberfläche angezogen werden bevor sie zu den Atmungsorganen, insbesondere zu den Nasenlöchern, gelangen.

Diese Aufgabe wird erfindungsgemäß dadurch gelöst, daß die nadelförmige Hochspannungselektrode in einem Abstand von einer mit dem zweiten Ausgang der Hochspannungsquelle verbundenen Gegenelektrode umgeben ist, die auf der gleichen Seite wie die nadelförmige Hochspannungselektrode aus dem Gehäuse vorsteht, so daß zwischen den beiden Elektroden ein elektrisches Feld gebildet wird, und daß das Gehäuse an einem elektrisch leitenden Tragelement angeordnet ist, das mit der Gegenelektrode elektrisch leitend verbunden ist und zur Kontaktierung des Körpers des Benutzers dient.

Durch die Anordnung der Gegenelektrode in der Nähe der nadelförmig ausgebildeten Hochspannungselektrode ergibt sich zwischen den beiden Elektroden ein elektrisches Feld, in welchem Partikel wie Staub. Pflanzen- und Blütenpollen und dergl. ionisiert werden. Durch das mit der Gegenelektrode elektrisch leitend verbundene Tragelement aus elektrisch leitendem Material befindet sich die Aussenoberfläche des Körpers der den erfindungsgemässen Ionenerzeuger tragenden Person auf dem elektrischen potential der Gegenelektrode, so dass die ionisierten Partikel von der Körperoberfläche der den Ionenerzeuger tragenden Person angezogen werden, bevor sie insbesondere durch die Nasenlöcher in den Nasenraum eindringen und dort Allergien hervorrufen können. Durch die nadelförmig ausgebildete Hochspannungselektrode und durch die die Hochspannungselektrode in einem Abstand umgebende Gegenelektrode ergibt sich ausserdem eine Richtungscharakteristik des elektrischen Feldes und somit der erzeugten Ionen.

Mit dem erfindungsgemässen Ionenerzeuger wird also nicht das Klima eines Raumes insgesamt verbessert, sondern gezielt die Umgebung der Atmungsorgane der einen solchen Ionenerzeuger benutzenden Person. Der Ionenerzeuger ist mit einer kontinuierlich Ionen erzeugenden Hochspannungsquelle ausgebildet, die von einem Akkumulator gespeist wird. Das tragbare Gerät kann dabei ein quaderförmiges Gehäuse mit derartigen Abmessungen aufweisen, dass das Gerät in einer Hand gehalten werden kann. Das Gehäuse des erfindungsgemässen Ionenerzeugers kann auch zylinderförmig oder scheibenförmig ausgebildet sein.

Das mit der Gegenelektrode elektrisch leitend verbundene Tragelement aus elektrisch leitendem Material kann z.B. als Ansteckclip oder as Anstecknadel ausgebildet sein.

Bei einer bevorzugten Ausführungsform des erfindungsgemässen Ionenerzeugers ist das Tragelement als Halskette ausgebildet. Auf diese Weise kann der Ionenerzeuger um den Hals getragen werden, so dass die nadelförmige Hochspannungselektrode und die Gegenelektrode in Richtung zu den Atmungsorganen, d.h. in Richtung zur Nase und zum Mund, weisen.

Ein weiterer Vorteil des erfindungsgemässen Ionenerzeugers besteht darin, dass die Hände während der Verwendung des Ionenerzeugers für andere Arbeiten frei bleiben, weil der Ionenerzeuger nicht mit einer Hand gehalten zu werden braucht.

Insbesondere bei einem Ionenerzeuger der zuletzt beschriebenen Art, d.h. bei einem Ionenerzeuger mit einer als Halskette ausgebildeten Befestigungseinrichtung, ist es vorteilhaft, das Tragelement und die beiden Elektroden des tragbaren Gerätes an der gleichen Seite des Gehäuses anzuordnen. Bei dieser Seite des Gehäuses handelt es sich vorzugsweise um die den Atmungsorganen zugewandte Oberseite des Gerätes, so dass das die Partikel ionisierende elektrische Feld zwischen den beiden Elektroden bis zu den Atmungsorganen reicht.

Es hat sich als zweckmässig erwiesen, dass die Gegenelektrode ringförmig ausgebildet ist und die nadelförmige Hochspannungselektrode konzentrisch umgibt. Durch die konzentrische Anordnung der ringförmig ausgebildeten Gegenelektrode ergibt sich bei eingeschaltetem Gerät zwischen den beiden Elektroden ein symmetrisches elektrisches Feld, so dass die Ionisierung von Partikeln, welche Allergien auslösen können, optimal ist, wobei die ionisierten Partikel nicht zu den Atmungsorganen gelangen sondern bereits vorher an die Aussenoberfläche der den Ionenerzeuger tragenden Person gelenkt werden.

Die ringförmige Gegenelektrode spannt dabei vorzugsweise eine Ebene auf, wobei die nadelförmige Hochspannungselektrode zu der von der Gegenelektrode aufgespannten Ebene senkrecht steht. Dabei weist die Gegenelektrode von dem Gehäuse vorzugsweise einen Abstand auf, der grösser ist als der Abstand der Spitze der nadelförmigen Hochspannungselektrode vom Gehäuse. Das bedeutet, dass die ringförmige Gegenelektrode in Richtung zur Spitze der sich verjüngenden nadelförmigen Hochspannugselektrode vor der Spitze der Hochspannungselektrode angeordnet ist. Durch eine derartige Ausbildung wird die nadelförmige Hochspannungselektrode gegen Berührungen geschützt, so dass eine durch die nadelförmige Hochspannungselektrode mögliche Verletzungsgefahr eliminiert ist.

Bei einer anderen Ausführungsform des erfindungsgemässen Ionenerzeugers sind die nadelförmige Hochspannungselektrode und die Gegenelektrode in einer Ausnehmung des Gehäuses derart angeordnet, dass sie aus der die Ausnehmung begrenzenden Oberfläche des Gehäuses nicht vorstehen. Auch bei dieser Ausbildung des Ionenerzeugers ergibt sich ausser einer optimalen Ionisierung von Partikeln durch die die nadelförmige Hochspannungselektrode konzentrisch umgebende ringförmige Gegenelektrode ein guter Berührungsschutz der nadelförmigen Hochspannungselektrode.

Es wurde gefunden, dass der erfindungsgemässe Ionenerzeuger zum Schutz von auf kleine Partikel, insbesondere auf pflanzliche Partikel allergisch reagierende Menschen gut verwendbar ist. Es hat sich beispielsweise gezeigt, dass Personen, die gegen Blütenpollen allergisch sind und auf solche Blütenpollen mit einem sog. Heuschnupfen reagieren, bei Verwendung eines erfindungsgemässen Ionenerzeugers gegen Pollenflug unempfindlich werden.

Weitere Einzelheiten, Merkmale und Vorteile ergeben sich aus der nachfolgenden Beschreibung zweier in der Zeichnung dargestellter Ausführungsbeispiele erfindungsgemässer Ionenerzeuger.

Es zeigen:

Figur 1 eine Vorderansicht eines Ionenerzeugers,

Figur 2 eine Draufsicht auf einen Ionenerzeuger gemäss Figur 1 in Blickrichtung II aus Figur 1,

Figur 3 eine andere Ausführungsform eines teilweise aufgeschnittenen Ionenerzeugers, und

Figur 4 eine Draufsicht auf einen Ionenerzeuger gemäss Figur 3 in Blickrichtung des Pfeiles IV in Figur 3.

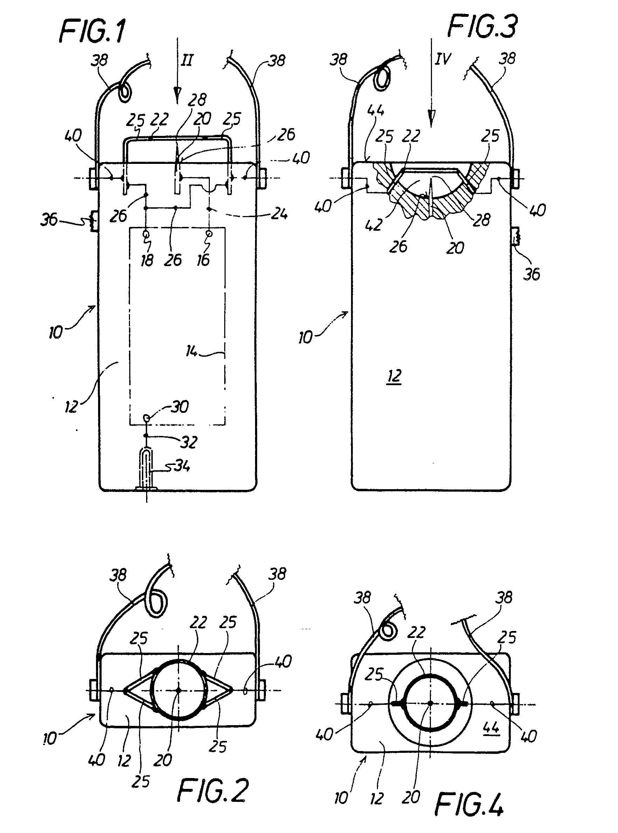

Die Figuren 1 und 2 zeigen einen Ionenerzeuger 10 der als tragbares Gerät ausgebildet ist. Der Ionenerzeuger 10 weist ein Gehäuse 12 auf, in welchem eine Gleichstrom-Hochspannungsquelle 14 angeordnet ist, die in Figur 1 strichliert angedeutet ist. Die Gleichstrom-Hochspannungsquelle 14 ist mit ihren Ausgängen 16 und 18 mit einer nadelförmigen Hochspannungselektrode 20, bzw. mit einer Gegenelektrode 22 elektrisch leitend verbunden. Die elektrisch leitende Verbindung zwischen den Ausgängen 16 und 18 und den beiden Elektroden 20 und 22 ist durch Anschlussdrähte 24 und 26 angedeutet.

Wie aus Figur 2 ersichtlich ist, umgibt die Gegenelektrode 22 die nadelförmige Hochspannungselektrode 20 konzentrisch. Die Gegenelektrode 22 ist ringförmig ausgebildet und mittels Haltegliedern 25 in einem Abstand vom Gehause 12 des Ionenerzeugers 10 angeordnet.

Wie aus Figur 1 ersichtlich ist, spannt die ringförmige Gegenelektrode 22 eine Ebene auf, die von der Basis 26 der nadelförmigen Hochspannungselektrode 20 einen grösseren Abstand aufweist als die Spitze 28 der Hochspannungselektrode. Auf diese Weise dient die Gegenelektrode 22 auch als Berührungsschutz, so dass Verletzungen, die durch die nadelförmige Hochspannungselektrode 20 möglich wären, eliminiert sind.

Mit der Bezugsziffer 30 ist ein Eingang der Gleichstrom-Hochspannungsquelle 14 gekennzeichnet, der über eine Verbindungsleitung 32 mit einer Anschlussbuchse 34 im Gehäuse 12 des Ionenerzeugers 12 elektrisch leitend verbunden ist. Mit Hilfe dieser Anschlussbuchse 34 ist es möglich, den in der Gleichstrom-Hochspannungsquelle 14 vorgesehenen — nicht dargestellten — Akkumulator im Bedarfsfalle wieder aufzuladen. Ein Schalter 36, der aus dem Gehäuse 12 des Ionenerzeugers ID vorsteht, dient zum Ein- bzw. Ausschalten des Ionenerzeugers.

Am Gehäuse ist ein Tragelement 38 in Form einer Halskette angeordnet, die in den Figuren nur abschnittweise angedeutet ist. Das Tragelement 38 besteht aus elektrisch leitendem Material und ist mittels Verbindungsleitungen 40 mit der Gegenelektrode 22 verbunden, so dass die Aussenoberfläche einer Person, die einen Ionenerzeuger um den Hals trägt, sich auf dem elektrischen Potential der Gegenelektrode befindet.

Wie aus Figur 1 deutlich ersichtlich ist, sind das Tragelement 38 in Form einer Halskette und die beiden Elektroden 20 und 22 des Ionenerzeugers 10 an der gleichen Seite des Gehäuses 12 angeordnet. Dadurch wird erreicht, dass das zwischen den beiden Elektroden erzeugte elektrische Feld bei um den Hals getragenem Ionenerzeuger zu den Atmungsorganen, d.h. zum Mund und zur Nase, ausgerichtet ist, so dass die Ionisierung von zu den Atmungsorganen strömenden Partikeln optimal ist. Die ionisierten Partikel werden dann an die Körperoberfläche angezogen, bevor sie in die Atmungsorgane, insbesondere in den Nasenraum, gelangen können.

Die Figuren 3 und 4 zeigen eine andere Ausführungsform eines erfindungsgemässen Ionenerzeugers 10 mit einem tragbaren quaderförmigen Gehäuse 12. Die im Gehäuse 12 angeordnete Gleichstrom-Hochspannungsquelle sowie die Verbindung der Gleichstrom-Hochspannungsquelle mit der Hochspannungselektrode 20 und der Gegenelektrode 22 ist in Figur 3 nicht dargestellt. Sie entspricht jedoch der in Figur 1 dargestellten Anordnung. Auch bei dieser Ausführungsform des erfindungsgemässen Ionenerzeugers 10 ist die Gegenelektrode 22 an Haltegliedern 25 derart gelagert, dass die ringförmige Gegenelektrode 22 die nadelförmige Hochspannungselektrode 20 konzentrisch umgibt. Die ringförmige Gegenelektrode 22 spannt auch bei dieser Ausführungsform eine Ebene auf, die von der Basis 26 der nadelförmigen Hochspannungselektrode 20 einen grösseren Abstand aufweist als die Spitze 28 der Hochspannungselektrode 20. Dadurch wird auch hier ein Berührungsschutz gewährleistet. Dem gleichen Zweck dient es, die beiden Elektroden 20 und 22 in einer Ausnehmung 42 des Gehäuses 12 derart anzuordnen, dass die Elektroden 29 und 22 aus der die Ausnehmung 42 begrenzenden Oberfläche 44 des Gehäuses 12 nicht vorstehen.

Mit der Bezugsziffer 38 ist auch in diesen Figuren ein abschnittweise dargestelltes Tragelement in Form einer Halskette bezeichnet. Die Verbindung zwichen dem Tragelement 38 und der Gegenelektrode 42 wird durch Verbindungsdrähte 40 hergestellt. Ein Schalter 36 dient zum Ein- und Ausschalten des erfindungsgemässen Ionenerzeugers.

Das Gehäuse 12 muss nicht quaderförmig sein, sondern es kann auch zylindrisch, scheibenförmig od. dgl. ausgebildet sein.

Die Gleichspannungs-Hochspannungsquelle 14 ist in den Figuren nicht dargestellt und auch nicht näher beschrieben worden, weil es sich dabei um eine an sich bekannte Hochspannungsquelle handelt, die eine Kaskadenschaltung aus Dioden und Kondensatoren aufweist.

## Patentansprüche

1. Tragbarer Ionenerzeuger (10) mit einer in einem Gehäuse (12) angeordneten Gleichspannungs-Hochspannungsquelle (14) mit zwei Ausgängen (16, 18), der eine aus dem Gehäuse vorstehende nadelförmige Hochspannungselektrode (20) aufweist, die elektrisch leitend mit einem Ausgang (16) verbunden ist, dadurch gekennzeichnet, daß die nadelförmige Hochspannungselektrode (20) in einem Abstand von einer mit dem zweiten Ausgang (18) der Hochspannungsquelle (14) verbundenen Gegenelektrode (22) umgeben ist, die auf der gleichen Seite wie die nadelförmige Hochspannungselektrode (20) aus dem Gehäuse (12) vorsteht, so daß zwischen den beiden Elektroden ein elektrisches Feld gebildet wird, und daß das Gehäuse (12) an einem elektrisch leitenden Tragelement (38) angeordnet ist, das mit der Gegenelektrode (22) elektrisch leitend verbunden ist, und zur Kontaktierung des Körpers des Benutzers dient.

2. Ionenerzeuger nach Anspruch 1, dadurch gekennzeichnet, daß das Tragelement (38) als Halskette ausgebildet ist.

3. Ionenerzeuger nach Anspruch 1, dadurch gekennzeichnet, dass das Tragelement (38) und die beiden Elektroden (20, 22) an der gleichen Seite des Gehäuses (12) angeordnet sind.

4. Ionenerzeuger nach Anspruch 1, dadurch gekennzeichnet, dass die Gegenelektrode (22) ringförmig ausgebildet ist und die nadelförmige Hochspannungselektrode (20) konzentrisch umgibt.

5. Ionenerzeuger nach Anspruch 4, dadurch gekennzeichnet, dass die ringförmige Gegenelektrode (22) eine Ebene aufspannt, wobei die nadelförmige Hochspannungselektrode (20) zu der von der Gegenelektrode (22) aufgespannten Ebene senkrecht ausgerichtet ist.

6. Ionenerzeuger nach Anspruch 5, dadurch gekennzeichnet, dass die Gegenelektrode (22) von dem Gehäuse (12) einen Abstand aufweist, der grösser ist als der Abstand der Spitze (28) der nadelförmigen Hochspannungselektrode (20) vom Gehäuse (12).

7. Ionenerzeuger nach einem der vorhergehenden Ansprüche, dadurch gekennzeichnet, dass die nadelförmige Hochspannungselektrode (20) und die Gegenelektrode (22) in einer Ausnehmung (42) des Gehäuses (12) derart angeordnet sind, dass sie aus der die Ausnehmung (42) begrenzenden Oberfläche (44) des Gehäuses (12) nicht vorstehen.

8. Verwendung eines Ionenerzeugers nach einem der vorhergehenden Ansprüche, zur Reduktion des Anteiles der in den Nasenraum einer Person eindringenden ionisierten Staubpartikel.

9. Verwendung eines Ionenerzeugers nach einem der Ansprüche 1 bis 7, zum Schutz von auf kleine Partikel, insbesondere auf pflanzliche partikel allergisch reagierende Personen.

## Revendications

1. Générateur d'ions, portatif, (10) comportant une source de haute tension continue (14) logée dans un boîtier (12), avec deux sorties (16, 18) et une électrode de haute tension (20) en forme d'aiguille, en saillie du boîtier, qui est reliée électriquement à la sortie (16), caractérisé en ce que l'électrode de haute tension (20) en forme d'aiguille est entourée à une certaine distance d'une contreélectrode (22) reliée à la seconde sortie (18) de la source de haute tension (14), contre-électrode qui est en saillie du boîtier (12) du même côté que l'électrode de haute tension (20) en forme d'aiguille, de manière qu'entre les deux électrodes, il se forme un champ électrique, et le boîtier (12) est relié à un élément de support (38) conducteur électrique qui est relié électriquement à la contre-électrode (22) et sert à faire le contact avec le corps de l'utilisateur.

2. Générateur d'ions selon la revendication 1, caractérisé en ce que l'élément de support (38) est en forme de chaînette.

3. Générateur d'ions selon la revendication 1, caractérisé en ce que l'élément de support (38) et les deux électrodes (20, 22) sont prévus du même côté du boîtier (12).

4. Générateur d'ions selon la revendication 1, caractérisé en ce que la contre-électrode (22) est de forme annulaire et entoure de manière concentrique l'électrode de haute tension (20) en forme d'aiguille.

5. Générateur d'ions selon la revendication 4, caractérisé en ce que la contre-électrode annulaire (22) sous-tend un plan et l'électrode de haute tension (20) en forme d'aiguille est perpendiculaire à ce plan sous-tendu par la contre-électrode (22).

6. Générateur d'ions selon la revendication 5, caractérisé en ce que la contre-électrode (22) est à une certaine distance du boîtier (12), distance qui est supérieure à la distance entre la pointe (28) de l'électrode de haute tension (20) en forme d'aiguille et le boîtier (12).

7. Générateur d'ions selon l'une des revendications précédentes, caractérisé en ce que l'électrode de haute tension (20 en forme d'aiguille et la contre-électrode (22) sont placées dans une cavité (42) du boîtier (12) de manière à ne pas être en saillie par rapport à la surface supérieure (44) du boîtier (12) qui délimite la cavité (42).

8. Application d'un générateur d'ions selon l'une des revendications précédentes pour réduire la fraction de particules de poussière ionisées pénétrant dans la cavité nasale d'une personne.

9. Application d'un générateur d'ions selon l'une des revendications 1 à 7 pour protéger des personnes allergiques à de petites particules notamment de particules végétales.

**Claims**

1 A portable ionizer (10) with a direct current-high voltage source (14) with two terminals (16, 18), having a needle-shaped high voltage electrode (20) projecting from the casing which is connected for electric conduction to one terminal (16), characterized in that the needle-shaped high voltage electrode (20) is surrounded at a distance by a counter electrode (22) connected to the second terminal (18) of the high voltage source (14), which counter electrode projects from the casing (12) on the same side as the needle-shaped high voltage electrode (20), so that an electric field is formed between the two electrodes, and in that the casing (12) is arranged on an electrically conducting carrying element (38), which is connected to the counter electrode (22) for electric conduction, and serves for contact with the user's body.

2. An ionizer according to claim 1, characterized in that the carrying element (38) is designed as a necklace.

3. An ionizer according to claim 1, characterized in that the carrying element (38) and the two electrodes (20, 22) are arranged on the same side of the casing (12).

4. An ionizer according to claim 1, characterized in that the counter electrode (22) has an annular shape and that it concentrically surrounds the needle-shaped high voltage electrode (20).

5. An ionizer according to claim 4, characterized in that the annular counter electrode (22) defines a plane, the needle-shaped high voltage electrode (20) being orientated perpendicular to the plane defined by the counter electrode (22).

6. An ionizer according to claim 5, characterized in that the counter electrode (22) is placed at a distance from the casing (12) which is greater than the distance of the point (28) of the needle-shaped high voltage electrode (20) from the casing (12).

7. An ionizer according to one of the preceding claims, characterized in that the needle-shaped high voltage electrode (20) and the counter electrode (22) are arranged in a recess (42) of the casing (12) in such a way that they do not project from the surface (44) of the casing (12), which surface delimits the recess (42).

8. Use of an ionizer according to one of the preceding claims, for reducing the proportion of the ionized dust particles penetrating into an individual's nasal region.

9. Use of an ionizer according to one of claims 1 to 7, for protecting individuals having allergic reactions to small particles. in particular vegetable particles.

FIG.1

FIG.3

FIG.2

FIG.4